# EUROPÄISCHE PATENTSCHRIFT

(11) **EP 0 529 408 B1**
(45) Veröffentlichungstag und Bekanntmachung des Hinweises auf die Patenterteilung: **20.12.1995**
(21) Anmeldenummer: 92113726.1
(22) Anmeldetag: 12.08.1992
(51) Int. Cl.: A61F 2/38

(54) **Kniegelenkendoprothese**
Knee endoprosthesis
Endoprothèse de genou

(30) Priorität: 24.08.1991 DE 4128171
(43) Veröffentlichungstag der Anmeldung: 03.03.1993
(73) Patentinhaber: AESCULAP AG, D-78501 Tuttlingen (DE)
(72) Erfinder: Blömer, Wilhelm, D-7200 Tuttlingen (DE); Schultz, Robert, D-7208 Spaichingen (DE)
(74) Vertreter: Hoeger, Stellrecht & Partner

(56) Entgegenhaltungen:
- EP-A- 0 186 471
- EP-A- 0 349 173
- WO-A-92/08424
- FR-A- 2 290 883

## Beschreibung

Die Erfindung betrifft eine Kniegelenkendoprothese mit einer Tibialagerfläche, einem auf dieser verschiebbaren Lagerkörper mit zwei konkav gekrümmten Lagerschalen, zur beweglichen Aufnahme eines Femurgelenkteils und mit einer Drehführung, die den Lagerkörper auf der Tibialagerfläche um eine senkrecht auf dieser stehende Drehachse führt.

Um die komplizierte Dreh- und Gleitbewegung eines Kniegelenkes bei einer Kniegelenkendoprothese soweit wie möglich nachzuahmen, ist es bekannt, auf einer ebenen Tibialagerfläche einen Lagerkörper frei verschieblich zu lagern, auf dem ein Femurgelenkteil verschieblich aufliegt. Bei einfachen Ausgestaltungen ist der Lagerkörper auf der ebenen Tibialagerfläche frei verschieblich und wird nur durch die die beiden Gelenkteile festlegenden Bänder in seiner Verschiebebewegung begrenzt (GB-PS 1 603 833), bei anderen Ausgestaltungen begrenzt ein aus der Tibialagerfläche nach oben vorstehender Vorsprung, der in eine Ausnehmung an der Unterseite des Lagerkörpers eingreift, diese Bewegung (US-PS 4 085 466). Es ist weiterhin bekannt, den Lagerkörper zu teilen und die beiden dabei entstehenden Lagerschalen auf der Tibialagerfläche längs einer gekrümmten Führung zu führen, die beispielsweise im Querschnitt schwalbenschwanzförmig ausgebildet sein kann (EP-B1-0 021 421). Diese zuletzt genannte Lagerung ermöglicht zwar eine exakte Führung der beiden Lagerkörperteile längs dieser Führung, die Konstruktion ist jedoch infolge der beiden Längsführungen relativ kompliziert und schränkt insbesondere die Drehbewegung des gesamten Gelenkes ein.

Es sind daneben auch Kniegelenkendoprothesen bekannt, die nur eine Rotation des Lagerkörpers relativ zur Tibialagerfläche ermöglichen, dagegen keine Transversalverschiebung (T.A.C.K. Knie der Firma Waldemark Link GmbH & Co., Hamburg, Prospekt 736 d-en/4.91). Bei dieser Kniegelenkprothese wird zwar ein relativ unkomplizierter Aufbau erreicht, da das Kniegelenk aber nur eine Rotation des Lagerkörpers zuläßt, kann die natürliche Bewegung nur teilweise nachgebildet werden.

In der EP 186 471 A3 ist eine Kniegelenkendoprothese beschrieben, bei welcher ein Lagerkörper auf der Tibialagerfläche um eine senkrecht auf dieser stehende Drehachse geführt wird. Diese Drehachse wird durch einen Zapfen gebildet, der seinerseits in einer Kunststoffhülse geführt wird. Der Lagerkörper kann bei dieser Prothese nur eine Rotationsbewegung ausführen. Ausgehend von dieser bekannten Kniegelenkendoprothese ist es Aufgabe der Erfindung, eine gattungsgemäße Kniegelenkendoprothese so auszubilden, daß sie bei möglichst naturgetreuem Bewegungsablauf trotzdem einen sehr einfachen konstruktiven Aufbau aufweist.

Dieser Aufgabe wird bei einer Kniegelenkendoprothese der eingangs beschriebenen Art erfindungsgemäß dadurch gelöst, daß die Drehführung einen auf der ebenen Tibialagerfläche angeordneten, an dieser um eine senkrecht zur Tibialagerfläche angeordnete Drehachse drehbar gelagerten, geradlinigen stabförmigen Lenker umfaßt, der zwei in paralleler Richtung verlaufende, seitliche Führungsflächen aufweist, und daß der Lagerkörper an seiner Unterseite eine den stabförmigen Lenker aufnehmende, nach unten offene Nut trägt, deren Seitenwände in paralleler Richtung verlaufende seitliche Führungsflächen des Lagerkörpers bilden, an denen die seitlichen Führungsfläche des Lenkers anliegen, und diesen bei einer parallel zum Lenker erfolgenden freien Verschiebung führen.

Bei einer solchen Ausgestaltung ergibt sich durch die freie Drehbarkeit des Lenkers auch eine freie Drehbarkeit des Lagerkörpers um die senkrecht zur Tibialagerfläche angeordnete Achse, außerdem ermöglicht der Lenker die freie Längsverschiebung des Lagerkörpers längs des Lenkers, so daß eine überlagerte Dreh-Verschiebungs-Bewegung möglich wird, wobei dies in einfacher Weise durch den Lenker erzielt wird.

Ein besonderer Vorteil dieser Ausgestaltung liegt auch darin, daß die Führung zwischen Lenker einerseits und Lagerkörper andererseits eine flächige Führung ist, so daß sich geringer Verschleiß einstellt.

Dadurch, daß der Lagerkörper an seiner Unterseite eine den Lenker aufnehmende Nut trägt, deren Seitenwände die seitlichen Führungsflächen des Lagerkörpers bilden und die, den Lenker aufnimmt, ergibt sich insgesamt keine Vergrösserung des Kniegelenkes, andererseits wird der Lenker durch die Aufnahme im Lagerkörper gegen die Körperumgebung geschützt, so daß auch im montierten Zustand nicht die Gefahr besteht, daß Körperteile die freie Beweglichkeit beeinträchtigen.

Bei einer bevorzugten Ausführungsform hintergreifen sich der Lenker und der Lagerkörper gegenseitig und nehmen dadurch senkrecht zur Tibialagerfläche eine feste Position zueinander ein. Dieses Hintergreifen kann beispielsweise durch eine schwalbenschwanzförmige Querschnittsform des Lenkers und der Nut oder durch eine T-förmige Querschnittsform erreicht werden.

Weiterhin können Anschläge vorgesehen sein, die die Längsverschiebung des Lagerkörpers relativ zu dem Lenker begrenzen.

Um die drehbare Lagerung des Lenkers zu erreichen, kann dieser an seiner Unterseite einen senkrecht nach unten abstehenden Lagerstift tragen, der in eine senkrechte Aufnahmebohrung in der Tibialagerfläche eingreift. Der Zusammenbau ist daher besonders einfach, da lediglich der Stift des Lenkers in die Lagerbohrung einzusetzen ist, anschließend kann der Lagerkörper auf die Tibialagerfläche aufgelegt werden, wobei der Lenker in die Nut an der Unterseite des Lagerkörpers eingreift.

Vorteilhaft ist es auch, wenn der Lenker auf der Tibialagerfläche aufliegt, so daß der Lenker zusammen mit dem Lagerkörper bei der Drehbewegung längs der Tibialagerfläche bewegt wird.

Die nachfolgende Beschreibung bevorzugter Ausführungsformen der Erfindung dient in Zusammenhang mit der Zeichnung der näheren Erläuterung. Es zeigen:
- Figur 1 :: eine perspektivische Ansicht einer Tibialagerfläche mit aufliegendem Lagerkörper;
- Figur 2 :: eine Draufsicht auf die Anordnung der Figur 1;
- Figur 3 :: eine Detailansicht des Lenkers zur Führung des Lagerkörpers auf der Tibialagerfläche;
- Figur 4 :: eine Ansicht ähnlich Figur 2 einer Ausführungsform mit Verschiebungsanschlag und
- Figur 5 :: eine Darstellung des Lenkers der Figur 4.

Die in der Zeichnung dargestellte Kniegelenkendoprothese umfaßt lediglich den unteren, mit der Tibia zu verbindenden Teil, nämlich eine ebene Tibialagerfläche 1, die mittels eines Stieles 2 auf eine entsprechend präparierte Tibiaoberseite aufsetzbar und in dieser verankerbar ist, und einen auf der Tibialagerfläche 1 angeordneten Lagerkörper 3 aus Kunststoff, beispielsweise aus Polyethylen. Nicht dargestellt ist der auf dem Lagerkörper 3 gestützte künstliche Femurgleitflächenersatz.

Der Lagerkörper 3 weist zwei nebeneinanderliegende Lagerschalen 4 für die Femurcondylen auf, diese Lagerschalen 4 sind über eine Brücke miteinander verbunden, so daß der Lagerkörper 3 insgesamt einstückig ausgebildet ist.

Er ist auf der ebenen Tibialagerfläche 1 mit seiner ebenen Unterseite 6 flächig aufgelegt und gegenüber der Tibialagerfläche 1 frei verschieblich.

An der Unterseite 6 weist der Lagerkörper 3 im Bereich seiner Brücke 5 eine sich längs der Mittellinie des Lagerkörpers 3 erstreckende, nach unten offene Nut 7 auf, in der ein stabförmiger Lenker 8 so angeordnet ist, daß dessen Seitenflächen 9, 10 flächig an den Seitenflächen 11, 12 der Nut 7 anliegen. Der Lenker 8 weist an seiner Unterseite einen nach unten abstehenden Lagerzapfen 13 auf, der in eine senkrechte Lagerbohrung 14 in der Tibialagerfläche 1 eintaucht. Die Lagerbohrung 14 kann mit der Längsachse des Stieles 2 zusammenfallen.

Der Lagerzapfen 13 liegt auf diese Weise flächig auf der Tibialagerfläche 1 auf und ist um die durch Lagerzapfen und Lagerbohrung definierte Schwenkachse verschwenkbar. Gleichzeitig ist der Lagerkörper 3 längs des Lenkers 8 verschiebbar, so daß insgesamt die freie Beweglichkeit des Lagerkörpers 3 auf der Tibialagerfläche 1 durch den Lenker 8 eingeschränkt ist. Der Lagerkörper 3 kann einmal zusammen mit dem Lenker 8 um die durch Lagerzapfen 13 und Lagerbohrung 14 gebildete Schwenkachse verschwenkt werden, zum anderen kann er sich längs des Lenkers 8 bewegen.

Diese komplexe Schwenk- und Translationsbewegung ist für alle in der Praxis auftretenden Bewegungen ausreichend und ermöglicht eine volle Nachbildung der natürlichen Beweglichkeit des Kniegelenkes.

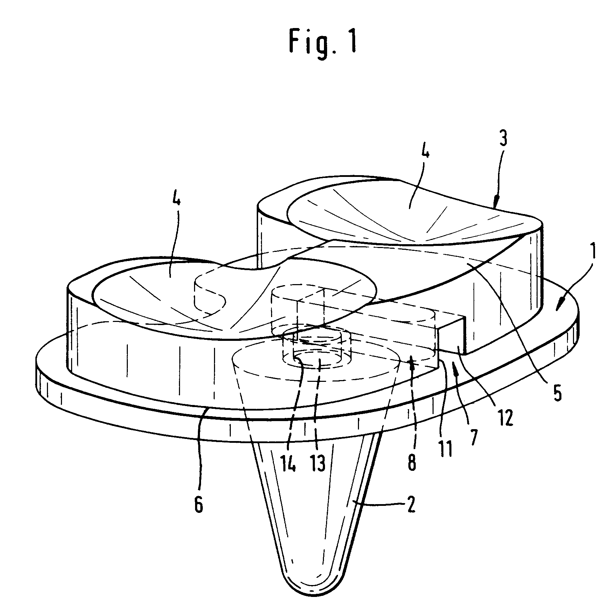

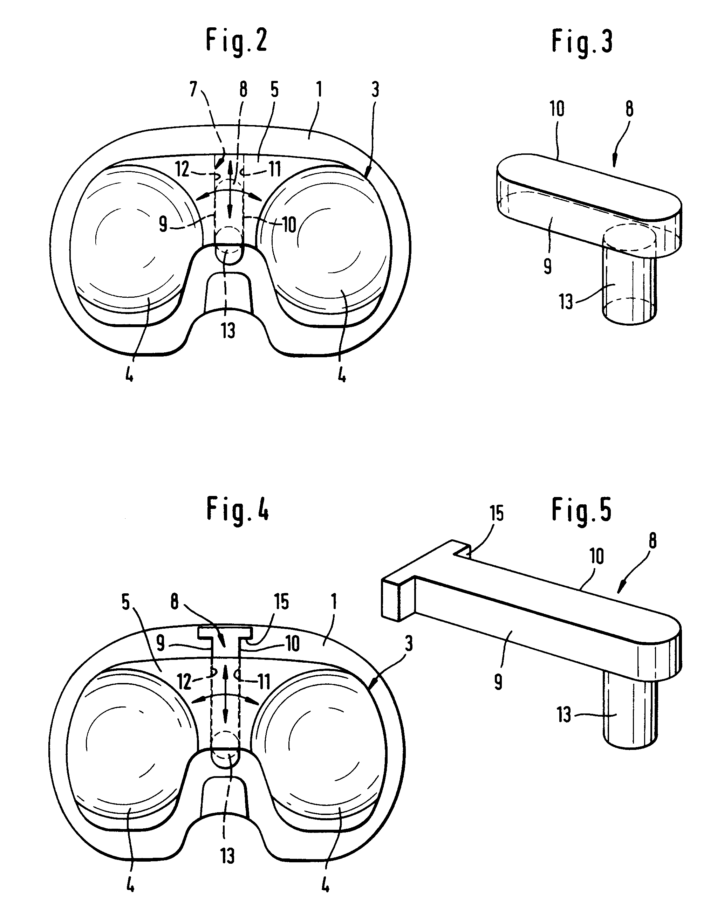

Das in den Figuren 4 und 5 dargestellte Ausführungsbeispiel unterscheidet sich lediglich dadurch von dem der Figuren 1 bis 3, daß der Lenker 8 an seinem freien Ende einen verbreiterten Anschlag 15 trägt, so daß die Längsbewegung des Lagerkörpers längs des Lenkers 8 begrenzt wird. Dabei kann der Lenker in der aus der Zeichnung ersichtlichen Weise so lang ausgebildet sein, daß er üblicherweise aus der Nut 7 hervorsteht. Es wäre aber auch möglich, den Lenker im Inneren des Lagerkörpers 3 anzuordnen und die Nut stufig zu erweitern.

## Patentansprüche

1. Kniegelenkendoprothese mit einer Tibialagerfläche (1), einem auf dieser verschiebbaren Lagerkörper (3) mit zwei konkav gekrümmten Lagerschalen zur beweglichen Aufnahme eines Femurgelenkteils und mit einer Drehführung, die den Lagerkörper (3) auf der Tibialagerfläche (1) um eine senkrecht auf dieser stehende Drehachse führt, dadurch gekennzeichnet, daß die Drehführung einen auf der ebenen Tibialagerfläche (1) angeordneten, an dieser um eine senkrecht zur Tibialagerfläche (1) angeordnete Drehachse drehbar gelagerten, geradlinigen stabförmigen Lenker (8) umfaßt, der zwei in paralleler Richtung verlaufende, seitliche Führungsflächen (9, 10) aufweist, und daß der Lagerkörper (3) an seiner Unterseite (6) eine den stabförmigen Lenker (8) aufnehmende, nach unten offene Nut (7) trägt, deren Seitenwände in paralleler Richtung verlaufende seitliche Führungsflächen (11, 12) des Lagerkörpers (3) bilden, an denen die seitlichen Führungsflächen (9, 10) des Lenkers (8) anliegen und diesen bei einer parallel zum Lenker (8) erfolgenden freien Verschiebung führen.

2. Prothese nach Anspruch 1, dadurch gekennzeichnet, daß der Lenker (8) und der Lagerkörper (3) sich gegenseitig hintergreifen und dadurch senkrecht zur Tibialagerfläche (1) eine feste Position zueinander einnehmen.

3. Prothese nach einem der voranstehenden Ansprüche, dadurch gekennzeichnet, daß Anschläge (15) vorgesehen sind, die die Längsverschiebung des Lagerkörpers (3) relativ zu dem Lenker (8) begrenzen.

4. Prothese nach einem der voranstehenden Ansprüche, dadurch gekennzeichnet, daß der Lenker (8) an seiner Unterseite einen senkrecht nach unten abstehenden Lagerzapfen (13) trägt, der in eine senkrechte Lagerbohrung (14) in der Tibialagerfläche (1) eingreift.

5. Prothese nach einem der voranstehenden Ansprüche, dadurch gekennzeichnet, daß der Lenker (8) auf der Tibialagerfläche (1) aufliegt.

## Claims

1. A knee-joint endoprosthesis, having a tibia bearing surface (1), a bearing body (3) which is displaceable on the latter and has two concavely curved bearing bowls to receive a femur joint part in a movable manner, and having a rotation guide which guides the bearing body (3) on the tibia bearing surface (1) about a rotational axis perpendicular to the latter, characterised in that the rotation guide comprises a rectilinear, bar-shaped guide rod (8) which is arranged on the plane tibia bearing surface (1), is mounted on the latter so as to be rotatable about a rotational axis arranged perpendicular to the tibia bearing surface (1) and has two parallel lateral guide surfaces (9, 10), and in that the underside (6) of the bearing body (3) bears a groove (7) which is open downwards, receives the bar-shaped guide rod (8) and whose lateral walls form parallel lateral guide surfaces (11, 12) of the bearing body (3), the lateral guide surfaces (9, 10) of the guide rod (8) contacting with the lateral guide surfaces (11, 12) of the bearing body (3) and guiding the guide rod (8) upon free displacement parallel thereto.

2. A prosthesis in accordance with Claim 1, characterised in that the guide rod (8) and the bearing body (3) engage behind one another and thereby assume a fixed position relative to one another perpendicular to the tibia bearing surface (1).

3. A prosthesis in accordance with any one of the preceding Claims, characterised in that stops (15) are provided which limit the longitudinal displacement of the bearing body (3) relative to the guide rod (8).

4. A prosthesis in accordance with any one of the preceding Claims, characterised in that the underside of the guide rod (8) bears a pivot pin (13) which projects perpendicularly downwards and engages into a perpendicular bearing bore (14) in the tibia bearing surface (1).

5. A prosthesis in accordance with any one of the preceding Claims, characterised in that the guide rod (8) rests on the tibia bearing surface (1).

## Revendications

1. Endoprothèse de genou, comportant une surface d'appui (1) de tibia, un corps de montage (3) pouvant être déplacé sur celle-ci et comprenant deux coques de montage incurvées de façon concave, pour la réception mobile d'une partie d'articulation du fémur, et comportant un guidage en rotation qui guide le corps de montage (3) sur la surface d'appui (1) de tibia autour d'un axe de rotation situé perpendiculairement sur celle-ci, caractérisée en ce que le guidage en rotation comprend un bras (8) linéaire et en forme de tige, agencé sur la surface d'appui plane (1) de tibia, qui est monté sur celle-ci en rotation autour d'un axe de rotation agencé perpendiculairement à la surface d'appui (1) de tibia, et qui présente deux surfaces de guidage latérales (9, 10) s'étendant en direction parallèle, et en ce que le corps de montage (3) présente sur sa face inférieure (6) une gorge (7) ouverte vers le bas et recevant le bras (8) en forme de tige, dont les parois latérales forment des surfaces de guidage latérales (11, 12) du corps de montage, qui s'étendent en direction parallèle et contre lesquelles s'appuient les surfaces de guidage latérales (9, 10) du bras, et qui guident celui-ci lors d'un déplacement libre qui s'effectue parallèlement au bras (8).

2. Prothèse selon la revendication 1, caractérisée en ce que le bras (8) et le corps de montage (3) s'engagent mutuellement par l'arrière et occupent ainsi une position fixe l'un par rapport à l'autre perpendiculairement à la surface d'appui (1) de tibia.

3. Prothèse selon l'une ou l'autre des revendications précédentes, caractérisée en ce qu'il est prévu des butées (15) qui limitent le déplacement longitudinal du corps de montage (3) par rapport au bras (8).

4. Prothèse selon l'une quelconque des revendications précédentes, caractérisée en ce que le bras (8) porte sur sa face inférieure un tenon de montage (13) faisant saillie perpendiculairement vers le bas, qui s'engage dans un perçage de montage perpendiculaire (14) dans la surface d'appui (1) de tibia.

5. Prothèse selon l'une quelconque des revendications précédentes, caractérisée en ce que le bras (8) repose sur la surface d'appui (1) de tibia.
